# EUROPÄISCHE PATENTSCHRIFT

(11) **EP 1 691 673 B1**
(45) Veröffentlichungstag und Bekanntmachung des Hinweises auf die Patenterteilung: **14.10.2009**
(21) Anmeldenummer: 03782097.4
(22) Anmeldetag: 19.11.2003
(51) Int. Cl.: A61B 5/00, A61B 1/04

(54) **VORRICHTUNG ZUR BILDGEBENDEN DIAGNOSE VON GEWEBE**
DEVICE FOR THE IMAGING DIAGNOSIS OF TISSUE
DISPOSITIF POUR LE DIAGNOSTIC PAR IMAGERIE D'UN TISSU

(43) Veröffentlichungstag der Anmeldung: 23.08.2006
(73) Patentinhaber: Richard Wolf GmbH, 75438 Knittlingen (DE)
(72) Erfinder: WEBER, Bernd-Claus, 76228 Karlsruhe (DE); DOLT, Martin, 75438 Knittlingen (DE); GOLL, Thomas, 75438 Knittlingen (DE); MÜLLER, Stefan, 75015 Bretten-Diedelsheim (DE); PEREIRA-DELGADO, Nicolas, 75433 Maulbronn (DE); VÖGELE, Michael, 75236 Kämpfelbach-Ersingen (DE); VAN DEN BERGH, Hubert, CH-1025 St-Sulpice (CH); WAGNIERES, Georges, CH-1095 Lutry (CH); GLANZMANN, Thomas, CH-4102 Binningen (CH); GABRECHT, Tanja, 58739 Wickede (DE)
(74) Vertreter: Hemmer, Arnd
(86) Internationale Anmeldenummer: PCT/DE2003/003828
(87) Internationale Veröffentlichungsnummer: WO 2005/051182

(56) Entgegenhaltungen:
- EP-A- 1 258 220
- WO-A-97/11636
- DE-A- 10 116 859
- DE-A- 10 201 005
- US-A- 5 827 190
- US-A- 6 110 106

## Beschreibung

Die Erfindung betrifft eine Vorrichtung zur bildgebenden Diagnose von Gewebe unter wahlweiser Anwendung von zwei Diagnosemethoden, nämlich einem Arbeitsmodus zur diagnostischen Weißlicht-Endoskopie DWLE und einem Arbeitsmodus zur diagnostischen Autofluoreszenz-Endoskopie DAFE, mit einer Lichtquelle, deren Strahlung über einen Lichtleiter und ein Endoskop zum Gewebe geleitet wird oder deren Leuchtmittel direkt an das Gewebe herangeführt werden kann, und mit einer Bildübertragungseinheit und einer Bildaufnahmeeinheit, welche an eine Bildverarbeitungseinheit angeschlossen ist, über welche ein Monitor mit einem Bildsignal versorgt wird. Die Bildübertragungseinheit kann aber auch direkt mit dem menschlichen Auge in Verbindung stehen. In diesem Fall kann auf die Bildaufnahmeeinheit, die Bildverarbeitungseinheit und den Monitor verzichtet werden.

Es ist bekannt, dass bei Anregung von Gewebe, beispielsweise von solchem der menschlichen Bronchialschleimhaut, mit Strahlung aus dem ultravioletten und / oder violetten und / oder blauen Spektralbereich dieses vor allen Dingen im Grünen und Roten fluoresziert **[1]**. Da diese Fluoreszenz ihren Ursprung in endogenen, also körpereigenen, Fluorophoren hat, spricht man auch von Autofluoreszenz. Beim gesunden Gewebe ist die Fluoreszenz im grünen Spektralbereich am stärksten, wohingegen diejenige im roten Spektralbereich weniger ausgeprägt ist.

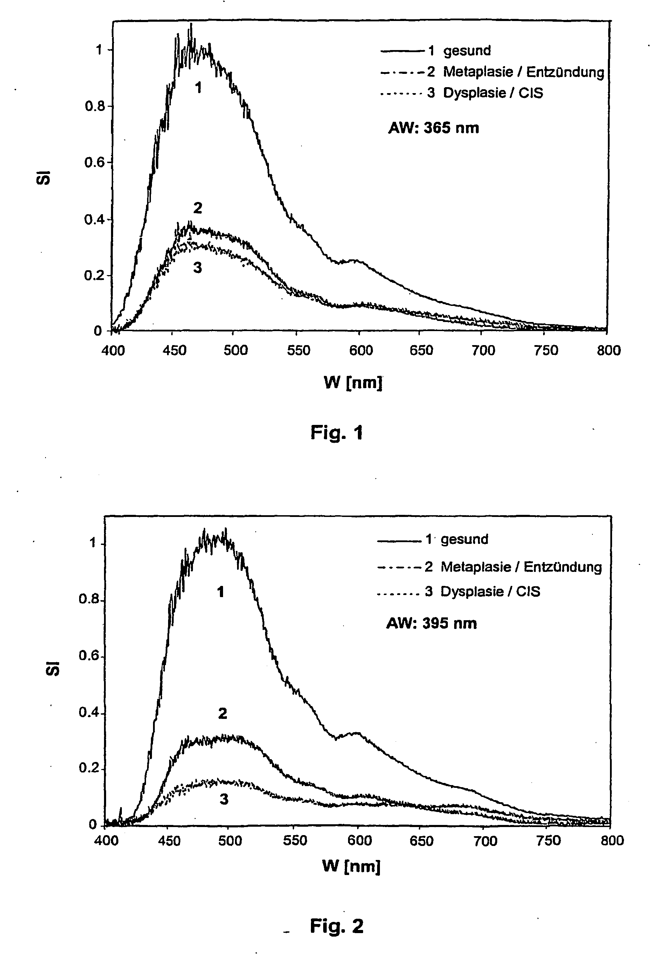

Mit zunehmender Atypie des Gewebes und zunehmendem Malignitätsgrad nimmt die Fluoreszenz jedoch ab, wobei der Rückgang in spektraler Hinsicht nicht gleichmäßig stattfindet, sondern wellenlängenabhängig ist: Der Rückgang im Grünen ist stärker ausgeprägt, während im Vergleich dazu die Abschwächung im Roten geringer ist. Dies wird auch aus den Fig. 1 bis 4 der Zeichnung.deutlich, welche typische spektrale Fluoreszenzintensitäten von menschlichem Bronchialgewebe für diverse Gewebezustände bei unterschiedlichen Fluoreszenz-Anregungswellenlängen AW, normiert auf das Fluoreszenzmaximum von gesundem Gewebe, zeigen und bei welchen die normierte spektrale Intensität SI über der Wellenlänge W in nm dargestellt ist. In den Figuren werden mit "Dysplasie" bzw. "CIS" (= Carcinoma in situ) intraepitheliale Läsionen bezeichnet, welche prä- bzw. frühmaligne Gewebeveränderungen darstellen. "Metaplasien" und "Entzündungen" gelten zwar gleichfalls als atypisches bzw. abnormales Gewebe, werden aber den nichtmalignen Gewebeveränderungen zugeordnet.

Im Rahmen der Krebsfrüherkennung mittels der diagnostischen Autofluoreszenz-Endoskopie DAFE können gemäß diesen Gesetzmäßigkeiten theoretisch zwei Effekte für eine Gewebedifferenzierung ausgenutzt werden: Zum einen erscheint atypisches Gewebe wesentlich dunkler als gesundes Gewebe, und zum anderen erfährt es eine Farbverschiebung vom Grünen zum Roten hin.

Die alleinige Sichtbarmachung und Beobachtung des ersten Effekts, also des Intensitätsrückgangs mit zunehmender Gewebe-Atypie, ist für eine optimale Gewebedifferenzierung nicht ausreichend, da auch gesundes Gewebe unter bestimmten Bedingungen - z. B. wenn es sich in vergleichsweise großem Abstand zum Endoskop befindet und / oder wenn die Gewebeoberflächen stark strukturiert sind und deshalb wie Lichtfallen wirken oder, allgemeiner formuliert, wenn die Rahmenbedingungen bei der endoskopischen Ausleuchtung und Bestrahlung des zu untersuchenden Gewebes nicht optimal sind - dunkel erscheinen kann und deshalb mit prä-, früh- und malignem Gewebe verwechselt werden kann. Das Resultat ist eine verminderte und deshalb suboptimale Spezifität bei der Detektion von prä-, früh- und malignen Läsionen.

Eine spezifitätssteigemde und damit wichtige Bedeutung kommt deshalb prinzipiell dem zweiten Effekt, also der Farbverschiebung vom Grünen zum Roten mit zunehmender Gewebe-Atypie, zu. Wird nämlich neben dem grünen Spektralbereich, in welchem der stärkste Fluoreszenzbeitrag und die ausgeprägtesten Intensitätsänderungen vorliegen, auch der rote Spektralbereich detektiert und im Bild farbgetreu wiedergegeben, dann hebt sich das atypische .Gewebe durch seine rötlich-braune Farbe von denjenigen Gewebepartien ab, welche unzureichend ausgeleuchtet und bestrahlt sind und welche sich deshalb nur dunkel, aber ohne nennenswerten Buntton darstellen.

Allerdings ist der praktische Nutzen bei dieser Vorgehensweise, der Detektion und Sichtbarmachung des gesamten spektralen Bereichs, in welchem Fluoreszenz auftritt, um damit zusätzlich die Farbverschiebung im Fluoreszenzbild mit zunehmender Gewebeabnormalität darzustellen, hinsichtlich der Spezifität für prä-, früh- und malignen Läsionen bescheiden und insbesondere bei bildgebenden Verfahren von untergeordneter Bedeutung. Dafür gibt es zwei Gründe:
Erstens ist die Gewebe-Fluoreszenz von atypischem Gewebe über den gesamten sichtbaren Spektralbereich stark reduziert. Atypisches Gewebe wird dementsprechend bei bildgebenden Verfahren gegenüber dem weitaus heller erscheinenden gesunden Gewebe in erster Linie als dunkel wahrgenommen. Die in den Spektren gut erkennbare Farbverschiebung (siehe Fig. 1 bis 4), wird auf dem Hintergrund der endlichen und begrenzten Helligkeit-Dynamik der Bildwiedergabeeinheit kaum wahrgenommen. Kommen außerdem konventionelle, d. h. hinsichtlich der DWLE optimierte Bildaufnahmeeinheiten zum Einsatz, was bei kombinierten DWLE/DAFE-Systemen im Hinblick auf ein gleichermaßen zu optimierendes DWLE-Bild anzustreben ist, ist die Farbverschiebung mit zunehmender Gewebeatypie bei der DAFE noch weniger wahrnehmbar, da in diesen Bildaufnahmeeinheiten in der Regel optische Filter eingesetzt werden, deren spektraler Transmissionsgrad im Langwelligen abnimmt, die also die roten Spektralanteile dämpfen, um eine Anpassung der Bildaufnahmeeinheit an die Hellempfindlichkeit des menschlichen Auges und damit eine optimale Farbwiedergabe bei der DWLE zu erzielen. Die ohnehin schwach ausgeprägten und für die Farbverschiebung verantwortlichen Rotanteile der Fluoreszenz erreichen deshalb die Bildaufnahmeeinheit nur in gedämpfter Form und spielen so im Bild nur eine untergeordnete Rolle.

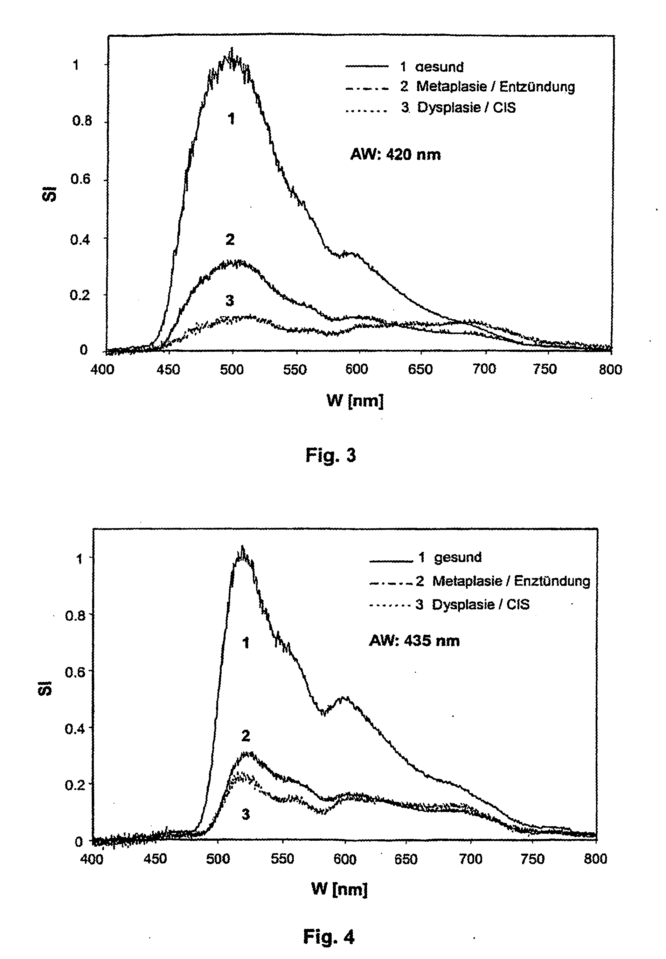

Zweitens ist die Farbverschiebung vom Grünen zum Roten mit zunehmender Gewebe-Atypie und damit der Gewebe-Farbkontrast im Sinne einer guten Gewebedifferenzierung nicht optimal aufgrund des mit zunehmendem Malignitätsgrad zwar weniger stark ausgeprägten, aber dennoch keinesfalls vernachlässigbaren Rückgangs der Rotfluoreszenz. Wäre beispielsweise die Rotfluoreszenz vom Grad der Gewebeatypie unabhängig, also diesbezüglich konstant, und bliebe sie beispielsweise außerdem hinsichtlich der Intensität auf dem vergleichsweise hohen Niveau, was bei gesundem Gewebe zu beobachten ist, siehe beispielsweise die spektralen Fluoreszenzintensitäten von gesundem Gewebe für Wellenlängen mit mehr als 600nm in Fig. 4, wäre bei bildgebenden Verfahren eine wesentlich deutlichere Farbveränderung mit zunehmender Gewebeatypie zu beobachten. Die Rotfluoreszenz würde dann als echte, d. h. hinsichtlich ihrer Intensität konstante Farbreferenz wirken: Beim gesunden Gewebe würde sie von dessen typischerweise stark ausgeprägter Grünfluoreszenz dominiert, weshalb dieses grün erscheinen würde, wie das auch tatsächlich beobachtet werden kann, aber umgekehrt würde sie, die Rotfluoreszenz, beim prä- und frühmalignen Gewebe die dort stark reduzierte Grünfluoreszenz im Gegensatz zur tatsächlich beobachtbaren Situation klar und deutlich dominieren, weshalb das prä- und frühmaligne Gewebe im Gegensatz zu den realen Verhältnissen erstens in einem sehr viel helleren und zweitens auch in einem sehr viel gesättigteren Rot erscheinen würde. Das prä- und frühmaligne Gewebe würde also nicht mehr länger in einem sehr dunklen, ungesättigt rötlich-braunen Farbton erscheinen, was hinsichtlich der Spezifität von prä- und frühmalignem Gewebe gegenüber unzureichend beleuchteten und bestrahlten Gewebepartien, also beispielsweise weit entfernten Gewebebereichen, von Vorteil wäre.

Desweiteren wird aus den Fig. 1 bis 4 deutlich, dass auf der einen Seite entzündetes Gewebe, also solches Gewebe, welches durch eine hohe Gefäßdichte gekennzeichnet ist, aber als nichtmaligne einzustufen ist, und auf der anderen Seite prä- und frühmalignes Gewebe für die meisten Fluoreszenz-Anregungswellenlängen AW ein in erster Näherung identisches Fluoreszenzverhalten aufweisen und deshalb bei einer bildgebenden Darstellungsweise, welche ausschließlich auf der Darstellung der Fluoreszenz des Gewebes beruht, praktisch kaum voneinander zu unterscheiden sind. Dies gilt insbesondere dann, wenn, wie das bei der Durchführung einer bildgebenden DAFE meist der Fall ist, die Referenz von großflächig umgebendem gesunden Gewebe gebildet wird, welches sich gegenüber beiden genannten Gruppen der Gewebeatypie deutlich abhebt: Das gesunde Gewebe erscheint, sofern keine modifizierende Bildverarbeitung vorgenommen wird, in einem vergleichsweise intensitätsstarken und deshalb hellen Grün, wohingegen einerseits entzündetes und andererseits prä- und frühmalignes Gewebe im Rahmen der endlichen und begrenzten Helligkeit-Dynamik der Bildaufnahmeeinheit vergleichbar dunkel und ähnlich braunrot erscheinen und deshalb, wenn dem gesunden Gewebe gegenübergestellt und auf dieses referenziert, kaum mehr voneinander zu unterscheiden sind. Erschwerend kommt noch hinzu, dass entzündetes Gewebe und prä- und frühmalignes Gewebe nicht immer direkt nebeneinander liegen und nicht unmittelbar miteinander verglichen werden können und außerdem das Fluoreszenzverhalten beider Gewebe-Zustände einer nicht vernachlässigbaren Streuung unterliegt, so dass eine Gewebeklassifizierung entsprechend den oben beschriebenen Gewebezuständen nicht in eindeutiger Weise vorgenommen werden kann.

Dies resultiert, zumindest bei den meisten Fluoreszenz-Anregungswellenlängen, in einer eingeschränkten und deshalb nicht optimalen Gewebedifferenzierung bei der DAFE. Jedoch müssen im Hinblick auf ausreichend helle Bilder bei der DAFE zusätzlich meist auch diese Wellenlängen für die Fluoreszenz-Anregung einbezogen werden.

Ziel muss deshalb die Realisierung einer Vorrichtung der eingangs genannten Art sein, welche im Modus der DAFE im Sinne einer klaren Unterscheidbarkeit zwischen einerseits prä-, früh- und malignem Gewebe, aber auch entzündetem Gewebe und Metaplasien, und andererseits solchem Gewebe, welches sich in einem größerem Abstand zum Endoskop befindet und / oder topologisch stark strukturiert ist und deshalb wie eine Lichtfalle wirkt und von dem deshalb nicht ausreichend viel Licht und Strahlung zurück an die Bildaufnahmeeinheit gelangt und über welches aus diesem Grund nicht genügend viel optische Information für eine Gewebebeurteilung und Zustandsbewertung vorliegt, den Schwerpunkt nicht auf die Detektion und Sichtbarmachung von Intensitätsunterschieden der Fluoreszenz legt. Entsprechend den obigen Ausführungen würde sonst atypisches Gewebe ähnlich dunkel wie das unzureichend ausgeleuchtete und bestrahlte Gewebe erscheinen und wäre im Modus der DAFE von letzterem nicht oder kaum zu unterscheiden, was in einer unzureichenden Spezifität resultieren würde.

Stattdessen soll bei der DAFE vielmehr eine Gewebedarstellung realisiert werden, bei welcher sich die diversen Formen der Gewebe-Atypie gegenüber dem unzureichend ausgeleuchteten und deshalb dunkel erscheinenden Gewebe in hellen Bunttönen darstellen. Dabei soll im Sinne einer optimierten Farbdifferenzierung und Farbkontrastierung entsprechend den obigen Erläuterungen erstens eine echte, d. h. im Hinblick auf die Intensität konstante Farbreferenz, geschaffen werden, also eine Farbreferenz, deren detektierte und im Bild wiedergegebene Intensität von allen genannten Gewebezuständen, also von Metaplasien, prä-, früh- und malignem Gewebe, insbesondere aber auch von entzündetem Gewebe, idealerweise völlig, zumindest aber in erster Näherung unabhängig ist, und zweitens soll die detektierte und im Bild wiedergegebene Intensität dieser Farbreferenz in der Größenordnung der im Bild wiedergegebenen Fluoreszenz-Intensität liegen.

Nur so gelingt eine Bildgebung, bei der im Modus der DAFE ausschließlich die entfernten und / oder die aufgrund ihrer Topologie wie Lichtfallen wirkenden Gewebepartien dunkel erscheinen und sich damit von allen Formen der Gewebeatypie, nämlich Metaplasien, prä-, früh- und malignen Gewebe, aber auch entzündetem Gewebe, von welchen aufgrund ausreichender Beleuchtung und Bestrahlung genügend Licht und Strahlung zurück an die Bildaufnahmeeinheit gelangen kann und insofern die gesamte, für die Vorgehensweise typische optische Information für eine Beurteilung des Gewebezustands vorliegt, eindeutig differenzieren lassen: Letztere erscheinen im Modus der DAFE dann in hellen Bunttönen.

Der Anwender weiß dann also, dass er bei den dunkel erscheinenden Gewebebereichen keine Aussage über deren Zustand machen kann und dass dort, gegebenenfalls durch Verkürzung des Abstandes Endoskop - Gewebefläche und damit durch eine verbesserte Ausleuchtung und Bestrahlung oder durch andere Maßnahmen, detailliertere optische Informationen eingeholt werden müssen. Der Anwender weiß dann aber auch, dass die sich in hellen Bunttönen darstellenden Gewebebereiche bereits ihr gesamtes optisches Potenzial für eine Zustandsbewertung bei der DAFE verfügbar gemacht haben und mit dieser Diagnosemethode, zumindest jedoch bei der dargestellten Vorgehensweise, keine weiteren Informationen zu erwarten sind.

Ziel sollte es im Sinne einer optimierten Gewebedifferenzierung bei der DAFE desweiteren sein, Gewebe, welches durch eine starke Vaskularisation oder Blutkonzentration gekennzeichnet ist, also beispielsweise entzündetes Gewebe, von prä-, früh- und malignem Gewebe, dessen Gefäßdichte deutlich geringer ist oder sogar zusätzlich von prä-, früh- und malignem Gewebe, das lokal eine ähnlich hohe Gefäßdichte aufweist, die jedoch in einem anderen Abstand zur Gewebeoberfläche auftritt, unterscheiden zu können.

Bekannt ist ein bildgebendes, kombiniertes DWLE / DAFE-System **[2]**, bei dem im Arbeitsmodus der DAFE ein kleiner Anteil des am Gewebe remittierten Fluoreszenz-Anregungslichtes aus dem langwelligen blauen Spektralbereich von der Bildaufnahmeeinheit detektiert wird und das daraus resultierende Blau-Remissionsbild dem eigentlichen, simultan erzeugten Fluoreszenzbild, welches sich in der Summe aller spektralen Fluoreszenzanteile bei gesundem Gewebe als helles Grün darstellt, überlagert wird. Konkret wird dies dadurch erreicht, dass sich das Transmissionsband des Fluoreszenz-Anregungsfilters, welches beim Wechsel vom Arbeitsmodus der DWLE in den Arbeitsmodus der DAFE in den Strahlengang der Lichtquelle eingeschwenkt wird, und das Transmissionsband des Fluoreszenz-Anregung-Blockfilters im Strahlengang vor der Bildaufnahmeeinheit in einem schmalen spektralen Bereich überschneiden. Dabei ist der Überlappungsbereich der beiden Filter so spezifiziert, dass das Bild von gesundem Gewebe von Fluoreszenzlicht dominiert wird und deshalb grün erscheint, während das Bild von prä- und frühmalignem Gewebe, welches durch eine deutlich verminderte Fluoreszenz charakterisiert ist, von am Gewebe remittiertem und dann von der Bildaufnahmeeinheit detektiertem langweiligen Blaulicht dominiert wird und deshalb blau erscheint. Gesundes Gewebe wird demnach grün dargestellt, prä- und frühmalignes Gewebe blau. Gewebe in größerem Abstand zum Endoskop oder Gewebe, welches aufgrund seiner Topologie wie eine Lichtfalle wirkt, erscheint in abgedunkelter Form.

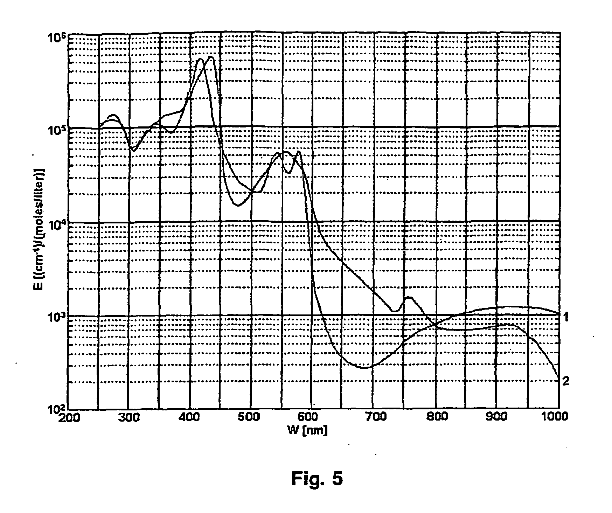

Allerdings erscheint bei dieser Vorgehensweise auch Gewebe, welches von einer hohen Gefäßdichte gekennzeichnet ist, bei der DAFE dunkel, und zwar auch dann, wenn sein Abstand zum Endoskop vergleichsweise klein und die Topologie unauffällig ist. Der Grund hierfür liegt in der vergleichsweise hohen Absorption von Strahlung aus dem nahen UV, dem violetten und dem blauen Spektralbereich durch Blut, also auch für Strahlung aus demjenigen Wellenlängenbereich, welcher für die Fluoreszenz-Anregung herangezogen wird und welcher bei diesem System für die Detektion und Sichtbarmachung von remittiertem Licht im DAFE-Modus herangezogen wird (siehe Fig. 5): Im Vergleich zum umgebenden, pathologisch unveränderten Gewebe absorbiert das von starker und dichter Gefäßbildung gekennzeichnete Gewebe einen hohen Anteil des violetten und blauen Fluoreszenz Anregungslichts. Nur noch ein vergleichsweise verminderter Anteil steht für die eigentliche Fluoreszenz-Anregung einerseits und für die Remission andererseits und somit für die Detektion durch die Bildaufnahmeeinheit bereit. Gewebe, welches durch eine starke Gefäßbildung charakterisiert ist, erscheint deshalb im DAFE-Modus wie Gewebe, welches sich in einem vergleichsweise großen Abstand zum Endoskop befindet oder welches aufgrund seiner topografischen Struktur wie eine Lichtfalle wirkt, nämlich abgedunkelt. Eine Differenzierung ist praktisch nicht möglich. Insgesamt ist damit die Gewebedifferenzierung im Arbeitsmodus der DAFE eingeschränkt.

In der Praxis kommt noch ein weiteres Problem hinzu:Auch entzündetes Gewebe ist durch eine hohe und dichte Gefäßbildung gekennzeichnet und verhält sich dementsprechend im Arbeitsmodus der DAFE in optischer Sicht in der oben beschriebenen Weise. Prä- und frühmalignes Gewebe ist jedoch oft auch gleichzeitig in entzündetem Zustand. Aufgrund der dann auftretenden verstärkten Absorption von violettem und blauem Licht durch die oberflächennahen Blutgefäße kann gemäß den obigen Erläuterungen nur noch vergleichsweise wenig blaues Licht remittiert und von der Bildaufnahmeeinheit detektiert werden. Diese entzündeten prä- und frühmalignen Läsionen erscheinen dann gleichfalls nur noch abgedunkelt und können nicht oder kaum mehr von entfernten oder wie Lichtfallen wirkenden Gewebepartien unterschieden werden. Damit sind bei der Krebsfrühdiagnose generell alle sich dunkel darstellenden Gewebepartien als verdächtig einzustufen, was die Spezifität enorm beeinträchtigt. Eine Differenzierung zwischen Gewebe mit hoher Gefäßdichte oder Blutkonzentration an der Gewebeoberfläche und Gewebe mit hoher Gefäßdichte dicht unter der Gewebeoberfläche ist gleichfalls nicht möglich, da das für die Detektion verwendete langwellige Blaulicht noch eine vergleichsweise hohe Eindringtiefe hat und deshalb in beiden Fällen ähnlich stark absorbiert wird und in beiden Fällen vergleichbar wenig Strahlung für die Rückstreuung zur Verfügung steht.

In der DE 102 01 005 A1 ist ein kombiniertes DWLE/DAFE-System beschrieben, welches im Modus der DAFE neben der vom Gewebe erzeugten Fluoreszenz-Strahlung zusätzlich am Gewebe remittierte Strahlung detektiert, welche entweder dem kurzwelligen oder dem langwelligen Ende des sichtbaren Spektralbereichs entstammt. Kriterium für die Auswahl des entsprechenden spektralen Bandes ist dabei, dass es als Farbreferenz dienlich ist im Sinne einer farblichen Unterscheidbarkeit zwischen einerseits gesundem Gewebe mit vergleichsweise großem Abstand zum Endoskop und/oder mit starker Oberflächenstrukturierung und andererseits prä-, früh- und malignem Gewebe. Ohne die Etablierung einer solchen Farbreferenz, also bei reiner Fluoreszenzdetektion, erscheinen gesundes Gewebe der beschriebenen Art und prä-, früh- und malignes Gewebe im Modus der DAFE nahezu gleich, nämlich dunkel, was in einer unzureichenden Spezifität resultiert. Durch zusätzliche Detektion von am Gewebe remittierter Strahlung aus einem Band der genannten Spektralbereiche im Modus der DAFE ist jedoch eine Unterscheidung der beiden Gewebetypen möglich.

Wird aber beispielsweise ausschließlich am Gewebe remittierte Strahlung aus dem roten Spektralbereich detektiert, ist es nicht oder kaum möglich, im Modus der DAFE prä-, früh- und malignes Gewebe von solchem nichtmalignem Gewebe, welches durch eine hohe Gefößdichte charakterisiert ist, zu unterscheiden. Mit dem beschriebenen Aufbau erscheinen beide Gewebezustände ähnlich rot, die Spezifität ist also suboptimal.

Wird ausschließlich am Gewebe remittierte Strahlung aus dem blauen Spektralbereich detektiert, entstehen die oben im Detail erläuterten Nachteile des in [2] beschriebenen Systems.

US 5,827,190 offenbart eine Vorrichtung sowie ein Verfahren für die Autofluoreszenzdiagnose, bei welcher neben der eigentlichen Gewebe-fluoreszenzstrahlung zusätzlich am Gewebe remittierte Strahlung aus zwei spektralen Bändern ausgewertet wird. Die Druckschrift offenbart eine farbliche Differenzierung zwischen normalem, entzündetem und abnormalem Gewebe. Allerdings können entzündetes und abnormales Gewebe nur voneinander unterschieden werden, wenn keine erhöhte Durchblutung oder Härnoglobinkonzentration vorliegt. Insofern ist es auch im Hinblick auf diesen Stand der Technik wünschenswert, eine weitere Gewebedifferenzierung zu ermöglichen.

Der Erfindung liegt daher die Aufgabe zu Grunde, eine Vorrichtung zur bildgebenden Diagnose von Gewebe, insbesondere für die Untersuchung des Bronchialbereichs, unter wahlweiser Anwendung der diagnostischen Weißlicht-Endoskopie DWLE und der diagnostischen Autofluoreszenz-Endoskopie DAFE zu schaffen, welche außerdem den eingangs genannten Zielen und Forderungen gerecht wird. Diese Vorrichtung soll vor allen Dingen dadurch gekennzeichnet sein, dass sie bei der DAFE eine fein differenzierte Gewebeerkennung gewährleistet.

Fein differenzierte Gewebeerkennung heißt schematisiert und vereinfacht, dass sich unterschiedliche Gewebezustände, nämlich erstens gesundes Gewebe, zweitens durch eine hohe Gefäßdichte charakterisiertes Gewebe, also beispielsweise entzündetes Gewebe, und drittens prä-, früh- und malignes Gewebe mit einer weniger stark ausgeprägten Vaskularisation, und gegebenenfalls viertens sogar prä-, früh- und malignes Gewebe mit einer lokal hohen Gefäßdichte, die aber in einem anderen Abstand zur Gewebeoberfläche auftritt als beispielsweise beim entzündeten Gewebe, optisch und dabei vor allen Dingen farblich deutlich voneinander unterscheiden lassen.

Fein differenzierte Gewebeerkennung heißt aber auch, dass solches Gewebe, welches aufgrund seiner Entfernung zum Endoskop und / oder aufgrund seiner außergewöhnlichen Topologie oder, ganz allgemein formuliert, aufgrund der ungünstigen Rahmenbedingungen bei der endoskopischen Ausleuchtung und Bestrahlung des zu untersuchenden Gewebes und welches deshalb im Hinblick auf seinen Zustand nicht eindeutig beurteilt werden kann, sich farblich deutlich vom anderen, gut ausgeleuchteten und bestrahlten Gewebe abhebt und dadurch als solches klar identifizierbar ist.

Der Anwender soll also auf diese Weise umgehend jene Gewebepartien erkennen, welche noch einer detaillierteren Untersuchung, also beispielsweise einer verbesserten Ausleuchtung, bedürfen, und von jenen unterscheiden können, über welche die DAFE bereits schon ihr ganzes Potenzial an optischen Informationen hinsichtlich des Gewebezustands verfügbar gemacht hat.

Eine diese Aufgabe lösende Vorrichtung ist im Anspruch 1 angegeben. Vorteilhafte Weiterbildungen einer solchen Vorrichtung ergeben sich aus den Unteransprüchen.

Bei der erfindungsgemäßen Vorrichtung ist die auf das menschliche Auge oder auf die Bildaufnahmeeinheit im Arbeitsmodus der DAFE neben dem im Gewebe erzeugten Fluoreszenzlicht oder Teilen des im Gewebe erzeugten Fluoreszenzlichtes zusätzlich von der Lichtquelle bereitgestellte und am Gewebe remittierte Strahlung aus mindestens zwei spektralen Bändern übertragbar, wobei die Strahlung aus einem ersten Band so gewählt ist, dass das Remissionsverhalten des Gewebes von den unterschiedlichen Gewebezuständen unabhängig oder aber zumindest in erster Näherung unabhängig ist, und die Strahlung aus einem zweiten Band so gewählt ist, dass Gewebe mit einer vergleichsweise hohen Gefäßdichte bzw. Blutkonzentration hinsichtlich der Remission von Strahlung aus diesem zweiten Band anders verhält als Gewebe mit einer im Vergleich dazu geringeren Gefäßdichte bzw. Blutkonzentration und/oder als Gewebe mit einer ähnlich hohen Gefäßdichte bzw. Blutkonzentration, welche aber in einem anderen Abstand zur Gewebeoberfläche auftritt.

Die erfindungsgemäße Vorrichtung zeichnet sich dadurch aus, dass die Bildaufnahmeeinheit im Arbeitsmodus der DAFE neben dem eigentlichen, durch Anregung im Gewebe erzeugten Fluoreszenzlicht zusätzlich von der Lichtquelle bereitgestellte und am Gewebe remittierte Strahlung aus einem ersten spektralen Band detektiert. Die Wahl dieses ersten spektralen Bandes soll erfindungsgemäß so erfolgen, dass das Remissionsverhalten für die Strahlung aus diesem ersten Band von den unterschiedlichen Gewebezuständen, insbesondere von gesundem Gewebe, von prä-, früh- und malignem Gewebe und auch von Gewebe, welches durch eine vergleichsweise hohe Gefäßdichte bzw. Blutkonzentration charakterisiert ist, und dabei insbesondere von entzündetem Gewebe, gleich oder zumindest in erster Näherung vergleichbar ist, d. h. der Anteil der remittierten Strahlung von den unterschiedlichen Gewebezuständen unabhängig oder zumindest in erster Näherung unabhängig ist. Ein spektrales Band wäre aber auch dann geeignet, wenn die Remission von entsprechender Strahlung für abnormales Gewebe, also für prä-, früh- und malignes Gewebe und für Gewebe mit hoher Gefäßdichte bzw. Blutkonzentration, also beispielsweise für entzündetes Gewebe, höher wäre als für gesundes Gewebe.

Desweiteren zeichnet sich die erfindungsgemäße Vorrichtung dadurch aus, dass die Strahlung aus diesem ersten spektralen Band mit der Vorrichtung bzw. mit den die Intensität der Strahlung manipulierenden Komponenten der Vorrichtung so abgestimmt ist, dass der am Gewebe remittierte und von der Bildaufnahmeeinheit detektierte Anteil in der Größenordnung der dort gleichzeitig detektierten Gewebe-Fluoreszenzstrahlung liegt. In einer beispielhaften Ausführungsform realisiert man diese Abstimmung so, dass bei gesundem Gewebe dieser am Gewebe remittierte und von der Bildaufnahmeeinheit detektierte Strahlungsanteil hinsichtlich der Intensität etwa halb so groß ist wie der Fluoreszenzanteil.

Idealerweise ist dieses erste spektrale Band hinsichtlich der Lage im Spektrum und hinsichtlich der spektralen Breite sowie hinsichtlich der Transmission so zu wählen bzw. sind gegebenenfalls zusätzliche Maßnahmen so zu treffen, dass sich evtl. überlagernde und vom Gewebezustand abhängige Fluoreszenzanteile aus diesem spektralen Bereich oder diesen spektralen Bereichen keinen nennenswerten Beitrag gegenüber dem vom Gewebezustand unabhängigen oder zumindest in erster Näherung unabhängigen Remissionsanteil leisten. Erfindungsgemäß beschränkt man sich auf ein einziges spektrales Band und legt dieses ins langwellige Sichtbare, und zwar dorthin, wo die Gewebefluoreszenz ohnehin vernachlässigbar gering ist, nämlich in einen Wellenlängernbereich um die Wellenlänge 670 nm +/- 10 nm (siehe auch unten beschriebenes Ausführungsbeispiel).

Nur mit dieser Vorgehensweise gelingt es, eine echte Farbreferenz zu schaffen. Bei der beispielhaften Ausführungsform wird gesundes Gewebe vom Fluoreszenzlicht dominiert, wohingegen abnormales Gewebe, also prä-, früh- und malignes Gewebe und durch eine hohe Gefäßdichte charakterisiertes Gewebe, von der am Gewebe remittierten Strahlung aus dem langwelligen Sichtbaren dominiert wird, da die detektierte Fluoreszenz von abnormalem Gewebe stark reduziert ist. Entsprechend der vorgenommenen Bildverarbeitung erscheinen dann normales Gewebe und abnormales Gewebe in deutlich voneinander unterscheidbaren, aber hellen Bunt- bzw. Farbtönen. Im einfachsten Fall, wenn nämlich durch die Bildaufnahmeeinheit und die Bildverarbeitungseinheit keine zusätzlichen Farbtransformationen vorgenommen werden, erscheint bei der beispielhaften Ausführungsform das gesunde Gewebe im hellen Grün des Fluoreszenzlichts und das abnormale Gewebe im hellen Rot des Remissionslichts. Es ist aber auch denkbar, dass den unterschiedlichen spektralen Bereichen, also dem detektierten und zur Gewebedifferenzierung herangezogenen spektralen Bereich der Fluoreszenzstrahlung einerseits und dem sich davon unterscheidenden spektralen Bereich der remittierten Strahlung aus dem ersten spektralen Band andererseits mittels Bildverarbeitung und/oder aber auch schon bereits durch entsprechende Spezifizierung der Bildaufnahmeeinheit andere Farben zugewiesen werden.

Entscheidend ist, dass mit dieser Vorgehensweise normales und abnormales Gewebe, welches ausreichend beleuchtet und bestrahlt wird, differenziert werden kann von solchem Gewebe, welches ungenügend beleuchtet und bestrahlt wird: Letzteres erscheint dunkel und ist damit deutlich zu unterscheiden von den hellen Färb- und Bunttönen des gut ausgeleuchteten Gewebes. Von diesem weiß der Anwender, dass er bereits die gesamte optische Information, die die DAFE vermitteln kann, hat. Bei den dunkel erscheinenden Gewebepartien hingegen weiß er, dass er (noch) keine Aussage über den Gewebezustand treffen kann.

Bei Verzicht auf die Bereitstellung und Detektion von Strahlung aus diesem ersten spektralen Band, also bei Verzicht auf die Schaffung einer echten Farbreferenz, würde abnormales Gewebe oder, wie in der bereits existierenden Lösung [2] oben im Detail beschrieben, zumindest bestimmte Formen von abnormalem Gewebe vergleichbar dunkel wie unzureichend ausgeleuchtetes Gewebe erscheinen und wäre von letzterem nicht oder zumindest kaum zu unterscheiden (siehe Fig. 1 bis 4). Die Spezifität der Vorrichtung wäre dadurch stark beeinträchtigt.

Die erfindungsgemäße Vorrichtung zeichnet sich des Weiteren dadurch aus, dass die Bildaufnahmeeinheit im Arbeitsmodus der DAFE zusätzlich von der Lichtquelle bereitgestellte und am Gewebe remittierte Strahlung aus einem zweiten spektralen Band detektiert.

Die Wahl dieses zweiten spektralen Bandes erfolgt erfindungsgemäß so, dass sich nichtmalignes abnormales Gewebe, welches durch eine vergleichsweise hohe Gefäßdichte charakterisiert ist, beispielsweise entzündetes Gewebe, hinsichtlich der Remission von Strahlung aus diesem zweiten spektralen Band deutlich anders verhält als prä-, früh- und malignes Gewebe mit einer im Vergleich dazu geringen Gefäßdichte oder sogar zusätzlich anders verhält als prä-, früh- und malignes Gewebe mit einer lokal ähnlich hohen Gefäßdichte, die aber in einem anderen Abstand zur Gewebeoberfläche auftritt als beispielsweise beim entzündeten Gewebe.

Das zweite spektrale Band wird in einen vergleichsweise spektral schmalen Wellenlängenbereich von maximal 10 nm Halbwertsbreite (FWHM) mit der zentralen Wellenlänge 415 nm verlagert (siehe auch unten beschriebenes Ausführungsbeispiel).

Des Weiteren ist die Strahlung aus diesem zweiten spektralen Band mit der erfindungsgemäßen Vorrichtung bzw. mit den die Intensität der Strahlung manipulierenden Komponenten der Vorrichtung so abgestimmt, dass der am Gewebe remittierte und von der Bildaufnahmeeinheit detektierte Anteil in der Größenordnung der dort gleichzeitig detektierten Gewebe-Fluoreszenzstrahlung und der am Gewebe remittierten und dort gleichzeitig detektierten Strahlung aus dem ersten spektralen Band liegt.

In einer beispielhaften Ausführungsform beschränkt man sich für beide remittierte Anteile auf Strahlung aus jeweils einem spektralen Band. Das erste Band verlagert man ins langwellige Sichtbare, wie bereits oben beschrieben, das zweite Band ins kurzwellige Sichtbare. Dort weist Blut eine vergleichsweise hohe Absorption auf (siehe Fig. 5), d. h. Strahlung aus diesem Wellenlängenbereich wird von Gewebe, welches von einer hohen Gefäßdichte bzw. hohen Blutkonzentration gekennzeichnet ist, stark absorbiert, so dass im Vergleich zum normalen Gewebe und zum prä-, früh- und malignen Gewebe, welches durch eine vergleichsweise geringe Vaskularisation charakterisiert ist bzw. eine vergleichsweise geringe Blutkonzentration aufweist, nur noch ein geringer Anteil für die Remission verfügbar ist. Dieser Effekt kann optimiert werden, wenn man das zweite Band spektral einengt und um die zentrale Wellenlänge 415 nm anordnet, da sich in diesem Wellenlängenbereich das Absorptionsmaximum von Blut befindet.

Außerdem ist bekannt, dass mit abnehmender Wellenlänge die Eindringtiefe von Strahlung aus dem Sichtbaren und den angrenzenden spektralen Bereichen in biologisches Gewebe abnimmt, so dass es durch die spektrale Einengung dieses zweiten Bandes und die Wahl dieser relativ kurzen zentralen Wellenlänge zusätzlich gelingt, Gewebetypen mit vergleichsweise hohen und miteinander vergleichbaren Gefäßdichten oder Blutkonzentrationen, die jedoch in unterschiedlichen Abständen zur Gewebeoberfläche auftreten, optisch zu differenzieren, also beispielsweise entzündetes Gewebe mit einer hohen Vaskularisation oder Blutkonzentration von einem bestimmten Typus von prä-, früh- und malignem Gewebe mit einer vergleichbar hohen Vaskularisation oder Blutkonzentration, die aber in einem anderen Abstand zur Gewebeoberfläche auftritt als beim entzündeten Gewebe. Bei den genannten Wellenlängen, die dem extrem kurzwelligen sichtbaren Spektralbereich entstammen, gelingt diese optische Differenzierung auch dann, wenn diese Abstände hoher Gefäßdichte bzw. Blutkonzentration vergleichsweise gering sind, denn es gilt: Je kürzer die Wellenlängen dieses zweiten spektralen Bandes sind, um so enger dürfen die Orte hoher Gefäßdichte bzw. Blutkonzentration bei diesen beiden unterschiedlichen Gewebetypen hinsichtlich ihres Abstandes zur Gewebeoberfläche beieinander liegen, um sie trotzdem noch optisch, nämlich anhand ihres Remissionsverhaltens, voneinander differenzieren zu können, um so besser wird also die diesbezügliche räumliche Auflösung.

Der vergleichsweise hohe Absorptionskoeffizient von Blut bei Wellenlängen um 420 nm führt dabei dazu, dass hohe Gefäßdichten und Blutkonzentrationen nahe an der Oberfläche einen relativ hohen Anteil der eintreffenden Strahlung dieses zweiten Bandes absorbieren und nur wenig zurückgestreut wird.

Die relativ geringe Eindringtiefe von Strahlung im Wellenlängenbereich um 420 nm in biologisches Gewebe führt dazu, dass der am Gewebe rückgestreute Anteil im Wesentlichen nur aus den oberflächennahen Bereichen stammen kann, weil bei diesen Wellenlängen kaum noch Strahlung in tiefere Gewebebereiche vordringen kann. Oder anders formuliert: Das optische Verhalten hinsichtlich der rückgestreuten Strahlung wird bei kurzen Wellenlängen fast ausschließlich von den oberflächennahen Bereichen bestimmt. Gewebe mit einer vergleichsweise höhen Gefäßdichte bzw. Blutkonzentration, welche sich jedoch nicht im Bereich der Gewebeoberfläche befindet, verhält sich im Hinblick auf die rückgestreute Strahlung bei diesen kurzen Wellenlängen dementsprechend wie Gewebe mit einer vergleichsweise geringen Gefäßdichte bzw. Blutkonzentration.

Beide Effekte zusammen, der hohe Absorptionskoeffizient von Blut und die geringe Gewebeeindringtiefe für Wellenlängen am Rande des kurzwelligen Sichtbaren, führen also dazu, dass es gelingt, Gewebe mit einer hohen Gefäßdichte bzw. Blutkonzentration an der Oberfläche, also beispielsweise entzündetes Gewebe, im Hinblick auf die Rückstreuung einerseits in optimierter Form von Gewebe mit normaler Vaskularisation und Blutkonzentration zu differenzieren, andererseits aber auch von solchem Gewebe zu differenzieren, welches eine ähnlich hohe Gefäßdichte hat, die aber nicht unmittelbar an der Gewebeoberfläche auftritt, also beispielsweise von prä-, früh- und malignem Gewebe, welches eine erhöhte Vaskularisation und Blutversorgung in tieferen Gewebebereichen aufweist.

Diese Form von Differenzierung beginnt sich zwar bereits schon bei anderen Wellenlängenbereichen bemerkbar zu machen, zum Beispiel im langwelligen Blauen, erfährt jedoch im Violetten, nämlich in einem Wellenlängenbereich um 420 nm, sein Optimum, da insbesondere der Absorptionskoeffizient von Blut dort extrem hohe Werte annimmt und auch die Eindringtiefe der Stahlung aus diesem Wellenlängenbereich bereits sehr gering ist.

Die Abstimmung zwischen der Strahlung aus dem ersten und zweiten Band mit der Vorrichtung bzw. mit den die Intensität bestimmenden Komponenten der Vorrichtung wird in dieser Ausführungsform derart realisiert, dass die maximal remittierten Strahlungsanteile aus den beiden Bändern hinsichtlich der Intensität etwa halb so groß sind wie der maximale Fluoreszenzonteil, also der Fluoreszenzanteil von gesundem Gewebe.

Durch die Bereitstellung und Detektion von Strahlung aus dem ersten spektralen Band gelingt es also, wie oben bereits im Detail erläutert, eine echte Farbreferenz zu schaffen, die es ermöglicht, normales und abnormales Gewebe in unterschiedlichen Farb- und Bunttönen darzustellen, jedoch solches Gewebe, welches nicht genügend ausgeleuchtet und bestrahlt wird und über welches deshalb keine Aussage über den Gewebezustand getroffen werden kann, farblich davon klar zu differenzieren. Durch die Bereitstellung und Detektion von Strahlung aus dem zweiten spektralen Band wird es zusätzlich möglich, abnormales Gewebe noch weiter zu differenzieren, nämlich solches nichtmalignes abnormales Gewebe, welches durch eine hohe Gefäßdichte gekennzeichnet ist, also beispielsweise entzündetes Gewebe, von prä-, früh- und malignem Gewebe, welches sich durch eine vergleichsweise geringe Gefäßdichte auszeichnet, oder sogar zusätzlich von prä-, früh- und malignem Gewebe, welches zwar eine ähnlich hohe Gefäßdichte aufweist, die aber in einem anderem Abstand zur Gewebeoberfläche auftritt, zu unterscheiden.

Bei der beispielhaften Ausführungsform wird gesundes Gewebe vom Fluoreszenzlicht dominiert, wohingegen prä-, früh- und malignes Gewebe von der am Gewebe remittierten Strahlung aus den beiden genannten spektralen Bändern, dem langwelligen Sichtbaren und dem kurzwelligen Sichtbaren, dominiert wird. Das durch starke Gefäßbildung gekennzeichnete nichtmaligne Gewebe hingegen, also beispielsweise entzündetes Gewebe, wird aufgrund der hohen Absorption von Strahlung aus dem kurzwelligen Sichtbaren lediglich von der am Gewebe remittierten Strahlung aus dem langwelligen Sichtbaren dominiert.

Entsprechend der vorgenommenen Bildverarbeitung erscheinen dann normales Gewebe, prä-, früh- und malignes Gewebe sowie nichtmalignes Gewebe mit einer hohen Gefäßdichte in deutlich voneinander unterscheidbaren, aber hellen Farb- und Bunttönen. Unzureichend ausgeleuchtetes und bestrahltes Gewebe, also beispielsweise ein Gewebebereich in größerer Entfernung, hebt sich dunkel davon ab.

Im einfachsten Fall, wenn nämlich durch die Bildaufnahmeeinheit und die Bildverarbeitungseinheit keine Farbtransformation vorgenommen wird, erscheint das gesunde Gewebe im hellen Grün des Fluoreszenzlichts, das prä-, früh- und maligne Gewebe violett bzw. purpurfarben, hervorgerufen durch die Überlagerung von remittierter Strahlung aus den beiden genannten spektralen Bändern einerseits und durch die stark reduzierte Gewebefluoreszenz andererseits, und das von starker Vaskularisation gekennzeichnete nichtmaligne Gewebe im hellen Rot des Remissionslichts aus dem langwelligen Sichtbaren, verursacht durch die starke Absorption und deshalb stark verminderte Remission von Strahlung aus dem kurzwelligen Sichtbaren sowie die stark reduzierte Fluoreszenz einerseits und die nichtbeeinträchtigte Remission von Strahlung aus dem langwelligen Sichtbaren andererseits.

Es ist aber auch denkbar, dass den unterschiedlichen spektralen Bereichen, also dem detektierten und zur Gewebedifferenzierung herangezogenen spektralen Bereich der Fluoreszenzstrahlung, dem sich davon unterscheidenden ersten spektralen Bereich der remittierten Strahlung und dem sich davon unterscheidenden zweiten spektralen Bereich der remittierten Strahlung mittels Bildverarbeitung und / oder durch die Spezifizierung der Bildaufnahmeeinheit andere Farben zugewiesen werden. Die unterschiedlichen Gewebezustände präsentieren sich dann in entsprechend anderen Farb- und Bunttönen. Unzureichend ausgeleuchtetes Gewebe hebt sich aber weiterhin durch seine dunkle Erscheinung deutlich davon ab.

Die von der Lichtquelle bereitgestellte, am Gewebe remittierte und von der Bildaufnahmeeinheit detektierte Strahlung kann aber auch dem Ultravioletten oder dem Infraroten entstammen, sofern sie den oben genannten Forderungen hinsichtlich der Wahl der spektralen Bänder gerecht wird und dem Anwender der Vorrichtung durch zusätzlich Farbtransformation sichtbar gemacht wird.

Es ist auch denkbar, dass die Beaufschlagung des Gewebes mit zusätzlicher Strahlung aus mindestens den beiden oben genannten unterschiedlichen spektralen Bändern bzw. Gruppen von spektralen Bändern nicht gleichzeitig mit der Beaufschlagung von Fluoreszenz-Anregungsstrahlung erfolgt, sondern dass die Beaufschlagung sequenziell, also zeitlich nacheinander, erfolgt. Anschließend werden entweder die Remissionsbilder aus den unterschiedlichen spektralen Bändern und das Fluoreszenzbild gleichzeitig nebeneinander oder zeitlich aufeinanderfolgend dargestellt oder aber mittels Bildverarbeitung in der Bildverarbeitungseinheit einander überlagert und in einem Bild dargestellt. Mischformen sind gleichermaßen denkbar.

Weitere vorteilhafte Merkmale der erfindungsgemäßen Vorrichtung ergeben sich aus der Beschreibung eines in der Zeichnung dargestellten Ausführungsbeispiels. In der Zeichnung zeigen:
- Fig. 1 bis 4: die typischen spektralen Fluoreszenzintensitäten von menschlichem Bronchialgewebe für unterschiedliche Gewebezustände bei unterschiedlichen Anregungswellenlängen AW, normiert auf das Fluoreszenzmaximum von gesundem Gewebe über der Wellenlänge W in Nanometer,
- Fig. 5: den Extingtionskoeffizienten E in (cm⁻¹)/(mol/Liter) als Maß für die Absorption von Oxyhämoglobin (Kurve 1) und Deoxyhämoglobin (Kurve 2) über der Wellenlänge W in Nanometer,
- Fig. 6: schematisch den Aufbau einer Diagnosevorrichtung nach der Erfindung,
- Fig.7: eine mögliche Ausführungsform einer Optikeinheit einer Lichtquelle,
- Fig. 8: schematisch eine mögliche Ausführungsform des spektralen Transmissionsverhaltens T eines Fluoreszenz-Anregungsfilters über der Wellenlänge W in Nanometer,
- Fig. 9: schematisch eine mögliche Ausführungsform des spektralen Transmissionsverhaltens T eines auf das Fluoreszenz-Anregungsfilter der Fig. 8 abgestimmten Blockfilters über der Wellenlänge W in Nanometer und
- Fig. 10: in der Übersicht und in schematischer Form die einzelnen Farbbeiträge für die diversen Gewebezustände und Bildsituationen und der daraus resultierende Farbeindruck.

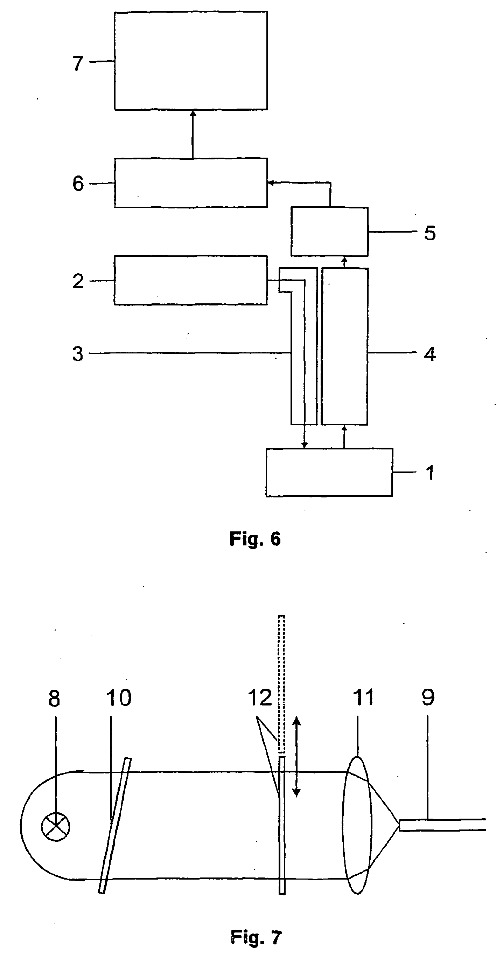

Fig. 6 zeigt in Form eines Blockbildes den Aufbau der Vorrichtung für die kombinierte diagnostische Weißlicht-Endoskopie DWLE und die diagnostische Autofluoreszenz-Endoskpie DAFE. Die Vorrichtung zur bildgebenden Diagnose des Gewebes 1 besteht aus einer Lichtquelle 2, welche im Arbeitsmodus der DWLE Weißlicht für die Beleuchtung und im Arbeitsmodus der DAFE Strahlung für die Fluoreszenz-Anregung, Strahlung aus einem ersten spektralen Band für die Schaffung einer echten Farbreferenz und Strahlung aus einem zweiten spektralen Band für eine weiterreichende Differenzierung von abnormalem Gewebe gemäß den obigen Ausführungen über einen Lichtleiter 3 an das Gewebe 1 heranführt. Der Lichtleiter 3 kann aus einer Einzelfaser, einem Faserbündel oder einem Flüssiglichtleiter oder auch aus einer Kombination dieser Elemente bestehen.

Sowohl das bei der DWLE fast ausschließlich durch remittiertes Licht erzeugte Bild als auch das bei der DAFE duch Fluoreszenzlicht erzeugte Bild, welchem zusätzlich remittierte Strahlung überlagert ist, wird über eine Bildübertragungseinheit 4 einer Bildaufnahmeeinheit 5 zugeführt, wo eine Umwandlung der optischen Signale in elektrische Signale stattfindet. Letztere werden an eine Bildverarbeitungseinheit 6 weitergeleitet, wo eine Aufbereitung der elektrischen Signale stattfindet, um beispielsweise ein Bild auf einem Monitor 7 zu erzeugen. Gleichermaßen ist aber auch die Aufzeichnung mit einem Videorecorder oder einer anderen videotechnischen Einrichtung möglich. Denkbar ist auch die endoskopische Direktbeobachtung, bei welcher die Bildaufnahmeeinheit 5, die Bildverarbeitungseinheit 6 und der Monitor 7 direkt durch den menschlichen Betrachter und sein Auge ersetzt werden.

Wird für die Bilddarstellung ein Videoendoskop, also ein sog. Chipendoskop, verwendet, bei welchem der Videochip im Endoskop selbst untergebracht ist, dann handelt es sich bei der Bildübertragungseinheit 4 im Wesentlichen um ein Objektiv, abhängig von der Konzeption des Videoendoskops aber gegebenenfalls auch um ein aufwändigeres Linsensystem, welches evtl. noch mit Bildfasern kombiniert ist, und bei der Bildaufnahmeeinheit 5 um das Sensorsystem des Videoendoskops. Die Bildverarbeitungseinheit 6 wird durch den zugehörigen Controller gebildet. Dabei handelt es sich sowohl beim Videoendoskop als auch beim Controller um Komponenten, die uneingeschränkt für die DWLE verwendet werden können.

Findet hingegen eine Kamera Verwendung, dann besteht die Bildübertragungseinheit 4 aus einem Endoskop-Objektiv, einem sich daran anschließenden Bildfaserbündel oder einem Linsen- oder Stablinsensystem, welche beide Teil eines Endoskops sind, aus einem Endoskop-Okular und evtl. einem Kamera-Objektiv. Diese Komponenten übertragen das Bild vom Gewebe auf die Bildaufnahmeeinheit 5. Letztere wird gebildet vom Sensorsystem eines Kamerakopfes. Bei der Bildverarbeitungseinheit 6 handelt es sich um den Kameratreiber. Auch bei der Kamera handelt es sich um ein Gerät, welches uneingeschränkt für die DWLE verwendet werden kann. Es kann sich sogar um eine konventionelle medizinische Kamera handeln.

Die im menschlichen Gewebe erzeugte und an dessen Oberfläche detektierbare Fluoreszenz, beispielsweise diejenige der Bronchialschleimhaut, ist größenordnungsmäßig um einen Faktor hundert geringer als die sich der Fluoreszenz überlagernde, am Gewebe remittierte Fluoreszenz-Anregungsstrahlung. Um deshalb die Gewebe-Fluoreszenz überhaupt erst sichtbar werden zu lassen, muß der zunächst im Bild dominierende Remissionsanteil völlig oder zumindest weitgehend eliminiert werden. Dies geschieht im Ausführungsbeispiel über ein Fluoreszenz-Anregungsstrahlung-Blockfilter. Dabei handelt es sich um ein optisches Filter, welches im Bildpfad vor der Bildaufnahmeeinheit 5, also beispielsweise in der Bildübertragungseinheit 4, untergebracht ist. Im einfachsten Fall kann es sich dabei um eine ohnehin sich im Bildpfad befindliche optische Komponente handeln, die mit entsprechenden optischen Schichten versehen ist. Das Fluoreszenz-Anregungsstrahlung-Blockfilter ist so konzipierf, dass seine Transmission im gesamten oder zumindest nahezu im gesamten spektralen Bereich, welcher für die Fluoreszenz-Anregung verfügbar gemacht wird, gegen Null geht. Außerhalb dieses spektralen Bereichs liegt die Transmission dieses Filters im Sichtbaren idealerweise bei hundert Prozent. Dieses Fluoreszenz-Anregungsstrahlung-Blockfilter ist in Fig. 6 nicht eingezeichnet.

Das oder die oder ausgewählte Leuchtmittel der Lichtquelle 2 können auch Teil des Endoskops sein. In dem Fall, dass es nur ein Leuchtmittel gibt und dieses im Endoskop untergebracht ist, kann auf den verlusterzeugenden Lichtleiter zwischen Lichtquelle und Endoskop verzichtet werden. Wird dieses Leuchtmittel am distalen Ende des Endoskops positioniert, kann sogar völlig auf Lichtleiter verzichtet werden. Im vorliegenden Ausführungsbeispiel wird vereinfachend von dem Fall ausgegangen, dass ein einziges Leuchtmittel Verwendung findet, welches in der Lichtquelle 2, also außerhalb des Endoskops untergebracht ist.

Fig. 7 zeigt die Optikeinheit der Lichtquelle 2 aus Fig. 6. Bei dem in dieser Lichtquelle verwendeten Leuchtmittel 8 handelt es sich um eine inkohärente, breitbandig im sichtbaren Spektralbereich emittierende Lampe. Wird bei dem idealerweise weißes Licht emittierenden Leuchtmittel eine Kurzbogenlampe verwendet, beispielsweise eine Xenonlampe, eine Mischgaslampe mit entprechend optimierter Gasmischung oder eine Lampe mit Quecksilberanteilen, gelingt die Strahlungseinkopplung in den Lichtleiter 9, welcher dem Lichtleiter 3 in Fig. 6 entspricht, besonders gut. Alternativ kann als Leuchtmittel auch eine Halogenlampe verwendet werden.

Im Ausführungsbeispiel der Fig. 7 findet eine Parabolspiegellampe Verwendung, welche ein paralleles Strahlenbündel abgibt. Ein sich fest im Strahlengang befindliches Filter 10 blockiert die UV- und die IRstrahlungsanteile des Leuchtmittels 8. Eine Linse 11 fokussiert das parallele Strahlenbündel des Leuchtmittels 8. Im Brennpunkt der Linse 11 befindet sich ein Lichtleiter 9, in welchen die gefilterte Strahlung des Leuchtmittels eingekoppelt wird.

Beim Umschalten in den Arbeitsmodus der DAFE wird das Fluoreszenz-Anregüngsfilter 12 in den parallelen Strahlengang des Leuchtmittels 8 eingeschwenkt. Dies geschieht beispielsweise über das Betätigen eines Fußschalters oder über einen Tastendruck an der Bedienfront der Lichtquelle. Beide Bedienelemente sind mit einer Steuereinheit verbunden, die den Filterschwenkmechanismus kontrolliert und die wiederum in der Lichtquelle untergebracht sein kann. Diese den Schwenkvorgang von Filter 12 betreffenden Komponenten sind in Fig. 7 nicht abgebildet. Beim Umschalten in den Arbeitsmodus der DWLE wird das Fluoreszenz-Anregungsfilter 12 aus dem Strahlengang ausgeschwenkt. Dies geschieht in der gleichen Weise wie das Einschwenken.

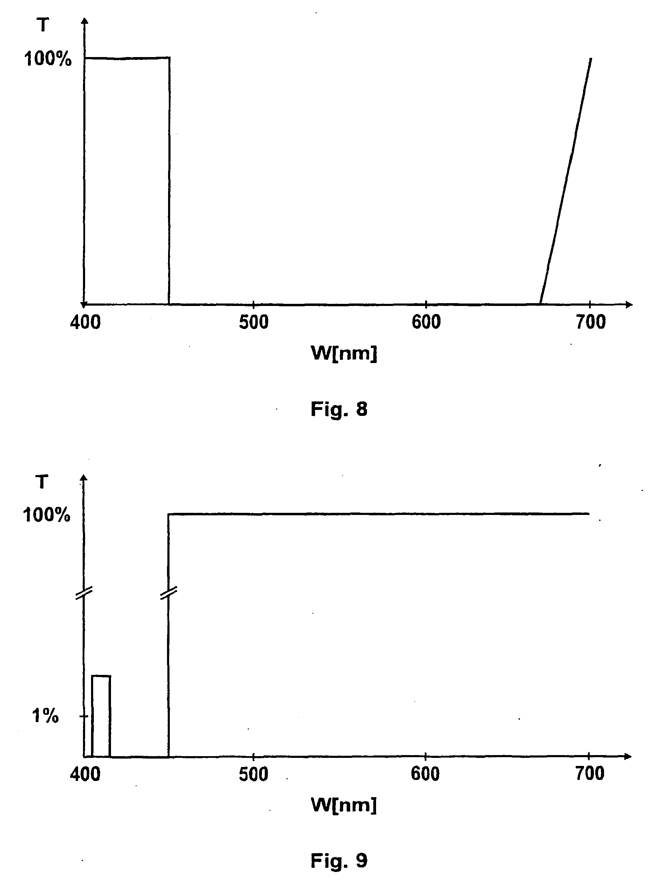

Fig. 8 zeigt eine mögliche Ausführungsform der spektralen Transmissionscharakteristik T des Fluoreszenz-Anregungsfilters 12 aus Fig. 7. Fig.9 zeigt eine mögliche Ausführungsform der spektralen Transmissionscharakteristik T eines auf das Fluoreszenz-Anregungsfilter 12 abgestimmten Fluoreszenz-Anregungsstrahlung-Blockfilters, welches wie oben beschrieben Teil der Übertragungseinheit 4 in Fig 6 sein kann, dort aber nicht explizit eingezeichnet ist.

Ein erstes Transmissionsband des Fluoreszenz-Anregungsfilters 12 liegt im Violetten und Blauen und dient zum einen der Fluoreszenz-Anregung. Der spektrale Transmissionsgrad geht idealerweise im gesamten Transmissionsband gegen hundert Prozent, um eine bestmögliche Fluoreszenz-Anregung im Sinne eines hellen. Fluoreszenzbildes zu erzeugen.

Entsprechend den vorausgehenden Überlegungen und im Sinne der Erfindung verfügt das Fluoreszenz-Anregungsfilter 12 über einen zweiten Transmissionsbereich, welcher in der hier beschriebenen Ausführungsform im langwelligen Sichtbaren, nämlich im langwelligen Roten liegt. Beispielsweise beginnt dieser zweite Transmissionsbereich bei einer Wellenlänge von etwa 665nm, liegt bei einer Wellenlänge von etwa 670nm im Prozentbereich und steigt idealerweise mit zunehmender Wellenlänge stark an. Entscheidend für die Wahl dieses Wellenlängenbereiches ist, dass die im Arbeitsmodus der DAFE an das Gewebe herangeführte und dort remittierte Strahlung aus diesem zweiten Transmissionsbereich des Fluoreszenz-Anregungsfilters vom Gewebezustand idealerweise völlig unabhängig, zumindest aber weitgehend unabhängig ist. Das bedeutet, dass gesundes Gewebe, prä-, früh- und malignes Gewebe, aber auch durch eine hohe Gefäßdichte charakterisiertes Gewebe, wie zum Beispiel entzündetes Gewebe, die Strahlung aus diesem zweiten Transmissionsbereich vergleichbar stark remittieren. Damit gelingt die Schaffung einer Farbreferenz, die gebildet wird von am Gewebe remittierter und von der Bildaufnahmeeinheit detektierter Strahlung aus diesem zweiten Transmissionsbereich des Fluoreszenz-Anregungsfilters. Abnormales Gewebe, welches bei der reinen Fluoreszenzdetektion, also ohne die zusätzliche Detektion von am Gewebe remittierter Strahlung, aufgrund vernachlässigbarer Fluoreszenz dunkel erscheinen würde und damit nicht unterschieden werden könnte von unzureichend beleuchtetem und bestrahltem Gewebe, erscheint aber so, zunächst ohne Betrachtung der nachfolgend weiter beschriebenen Maßnahmen, in der Farbe, welche durch die Strahlung aus diesem zweiten Transmissionsbereich bestimmt wird. Damit gelingt im Arbeitsmodus der DAFE eine klare Differenzierung von abnormalem Gewebe und unzureichend beleuchtetem und bestrahltem Gewebe.

Da die Transmission des Fluoreszenz-Anregungsstrahlung-Blockfilters in diesem spektralen Bereich idealerweise gegen hundert Prozent geht (siehe Fig 9), kann die am Gewebe remittierte Strahlung aus diesem spektralen Bereich von der Bildaufnahmeeinheit 5 zunächst in vollem Umfang detektiert werden. Zusätzliche sich im Strahlengang befindliche, die Transmission manipulierende optische Komponenten oder eine Kombination aus optischen und elektronischen Komponenten, die in den Abbildungen nicht eingezeichnet sind, sorgen jedoch dafür, dass im Ausführungsbeispiel der Anteil des Bildsignals, welcher diesem zweiten Transmissionsbereich des Fluoreszenz-Anregungsfilters entstammt, etwa halb so hoch ist wie das Bildsignal, welches seinen Ursprung in der Fluoreszenzstrahlung von gesundem Gewebe hat. So findet beispielsweise in der Bildaufnahmeeinheit 5 eine Sensoreinheit Verwendung, die im Hinblick auf eine gute Farbwiedergabe bei der DWLE optimiert ist. Wie bereits an anderer Stelle angedeutet ist deshalb der eigentlichen Detektoreinheit ein optisches Filter vorgelagert, welches diejenigen Strahlungsanteile, welche dem roten Spektralbereich entstammen, stark dämpft.

Dies hat unter anderem auch den Vorteil, dass die sich dem Remissionsanteil aus dem Roten überlagernden und vom Gewebezustand abhängigen Rotfluoreszenzanteile praktisch keine Rolle mehr spielen. Dadurch ist der Gesamt-Rotanteil im Bild, welcher sich zusammensetzt aus am Gewebe remittiertem Rotlicht und Gewebe-Rotfluoreszenzlicht, vom Gewebezustand praktisch unabhängig, und der Rotanteil im Bild kann als echte, d. h. konstante Farbreferenz betrachtet werden.

Entsprechend den vorausgehenden Überlegungen und im Sinne der Erfindung verfügt das Fluoreszenz-Anregungsstrahlung-Blockfilter neben dem eigentlichen ersten, spektral breiten Transmissionsbereich, welcher nahezu den gesamten sichtbaren Spektralbereich umfaßt, über einen zweiten Transmissionsbereich. Dieser liegt im Ausführungsbeispiel im Kurzwelligen (siehe Fig. 9). Durch entsprechende Abstimmung mit dem Fluoreszenz-Anregungsfilter 12 kann aber theoretisch dieser zweite Transmissionsbereich auch Teil des ersten, spektral breiten Transmissionsbereichs sein.

Entscheidend für die Wahl dieses Wellenlängenbereiches ist, dass nichtmalignes Gewebe, welches durch eine starke Vaskularisation gekennzeichnet ist, also beispielsweise entzündetes Gewebe, für Strahlung aus diesem spektralen Bereich ein deutlich anderes Remissionsverhalten aufweist als prä-, früh- und malignes Gewebe, welches durch eine deutlich geringere Gefäßdichte charakterisiert ist. Im Hinblick auf eine noch weiter verbesserte Gewebedifferenzierung ist der zweite Wellenlängenbereich sogar so zu wählen, dass beispielsweise entzündetes Gewebe mit einer erhöhten Gefäßdichte und Blutkonzentration direkt an der Gewebeoberfläche für Strahlung aus diesem spektralen Bereich außerdem ein deutlich anderes Remissionsverhalten aufweist als prä-, früh- und malignes Gewebe, welches eine vergleichbar hohe Gefäßdichte aufweist, die aber in einem anderen Abstand zur Gewebeoberfläche auftritt und idealerweise sogar in einem nur geringen Abstand zur Gewebeoberfläche auftreten kann.

Dieser zweite Transmissionsbereich des Fluoreszenz-Anregungsstrahlung-Blockfilters ist deshalb in einem spektralen Bereich plaziert, in welchem Blut eine verhältnismäßig starke Absorption aufweist, also beispielsweise im nahen UV und/oder im Violetten und/oder im Blauen (siehe Fig. 5). Dies führt dazu, dass Gewebe mit einer hohen Gefäßdichte und Blutkonzentration, wie zum Beispiel entzündetes Gewebe, einen bedeutend höheren Anteil der eintreffenden Strahlung aus diesem zweiten Transmissionsbereich des Fluoreszenz-Anregung-Blockfilters absorbiert und konsequenterweise ein sehr viel geringerer Anteil für die Remission zur Verfügung steht als bei prä-, früh- und malignem Gewebe mit einer geringeren Gefäßdichte.

Im Hinblick auf die erwähnte, noch weiter verbesserte Gewebedifferenzierung ist dieser zweite Transmissionsbereich des Fluoreszenz-Anregungsstrahlung-Blockfilters außerdem in und um einen spektralen Bereich platziert, in welchem die Blutabsorption ihren Maximalwert erreicht und außerdem die Gewebeeindringtiefe von entsprechender Strahlung in biologisches Gewebe bereits vergleichsweise gering ist. Dies führt dazu, dass erstens Gewebe mit hoher Gefäßdichte bzw. Blutkonzentration von Gewebe mit normaler Gefäßdichte bzw. Blutkonzentration in optimaler Weise differenziert werden kann und zweitens, dass zusätzlich auch noch Gewebe mit hoher Gefäßdichte bzw. Blutkonzentration in Bereich der Gewebeoberfläche, zum Beispiel entzündetes Gewebe, auch von Gewebe mit ähnlicher Vaskularisation unterschieden werden kann, wenn diese nur in einer gewissen Entfernung von der Gewebeoberfläche auftritt. Durch die Wahl eines extrem kurzwelligen Bereiches und der damit einhergehenden geringen Eindringtiefe der Strahlung kann dieser Abstand zur Gewebeoberfläche vergleichsweise gering sein. Es ist damit also möglich, beispielsweise Entzündungen mit vergleichsweise höher Gefäßdichte im oberflächennahen Bereich von bestimmten Formen von prä-, früh- und malignem Gewebe, welche gleichfalls eine erhöhte Gefäßdichte aufweisen, mittels der unterschiedlich stark remittierten Strahlung aus diesem zweiten spektralen Band und damit optisch zu unterscheiden, wenn sich diese erhöhte Gefäßdichte nur in einem der Eindringtiefe der Strahlung entsprechenden Mindestabstand von der Gewebeoberfläche befindet.

Beispielswiese hat dieser zweite Transmissionsbereich des Fluoreszenz-Anregung-Blockfilters seine zentrale Lage bei 415 nm, eine Halbwertsbreite von etwa 10nm und einen maximalen Transmissionsgrad im Prozentebereich (siehe Fig. 9).

Die spektrale Breite des Transmissionsbereiches und die Höhe des Transmissionsgrades einerseits, gegebenenfalls aber auch zusätzliche sich im Strahlengang befindliche, die Transmission manipulierende optische Komponenten oder eine Kombination aus optischen und elektronischen Komponenten andererseits, die in den Figuren nicht eingezeichnet sind, sorgen dafür, dass im Ausführungsbeispiel der Anteil des Bildsignals, welcher diesem zweiten Transmissionsbereich des Blockfilters entstammt und welcher von gesundem Gewebe oder prä-, früh- und malignem Gewebe der oben beschriebnen Art herrührt, etwa halb so hoch ist wie das Bildsignal, welches seinen Ursprung in der Fluoreszenzstrahlung von gesundem Gewebe hat.

Entsprechend den obigen Beschreibungen ergibt sich für die beispielhafte Ausführungsform im Arbeitsmodus der DAFE folgende Form der Gewebe-Darstellung: Gesundes Gewebe wird klar von Fluoreszenzlicht dominiert, dessen Hauptanteil dem Grünen entstammt. Die verbleibenden Fluoreszenzanteile aus dem Langwelligen und Kurzwelligen ergeben gemäß den Gesetzen der additiven Farbmischung im Wesentlichen wieder Grün. Da die jeweiligen Remissionsanteile aus dem langwelligen und kurzwelligen spektralen Bereich von klar geringerer Intensität sind, bleibt der resultierende Farbeindruck bei gesundem Gewebe grünlich.

Abnormales Gewebe, also sowohl die von überdurchschnittlich hoher Gefäßdichte gekennzeichneten Partien als auch die malignen Gewebebereiche und deren Vorstufen, ist generell durch eine stark reduzierte Fluoreszenz gekennzeichnet. Grün spielt dementsprechend bei dessen Farbdarstellung im Arbeitsmodus der DAFE eine vernachlässigbare Rolle.

Bei prä-, früh- und malignen Gewebe, welches keine überdurchschnittlich hohe Gefäßdichte aufweist, ist das Remissionsverhalten für die Strahlungsanteile aus dem Langwelligen und aus dem Kurzwelligen mit dem gesunden Gewebe vergleichbar, und die beiden Strahlungsanteile werden deshalb mit vergleichbarem Gewicht zur resultierenden Farbe beitragen. Prä-, früh- und malignes Gewebe wird somit Violett bzw. Purpur erscheinen.

Demgegenüber wird beim Gewebe, welches eine hohe Vaskularisation an der Oberfläche aufweist, kaum mehr Strahlung aus dem Kurzwelligen für die Remission verfügbar sein. Der dominierende und deshalb farbgebende Strahlungsanteil ist somit das Langwellige, der resultierende Farbeindruck ist Rot.

Bei Gewebe, welches unzureichend ausgeleuchtet ist, kann weder Fluoreszenzlicht noch Remissionsstrahlung in nennenswertem Umfang erzeugt werden oder den Detektor erreichen. Dieses Gewebe erscheint deshalb dunkel.

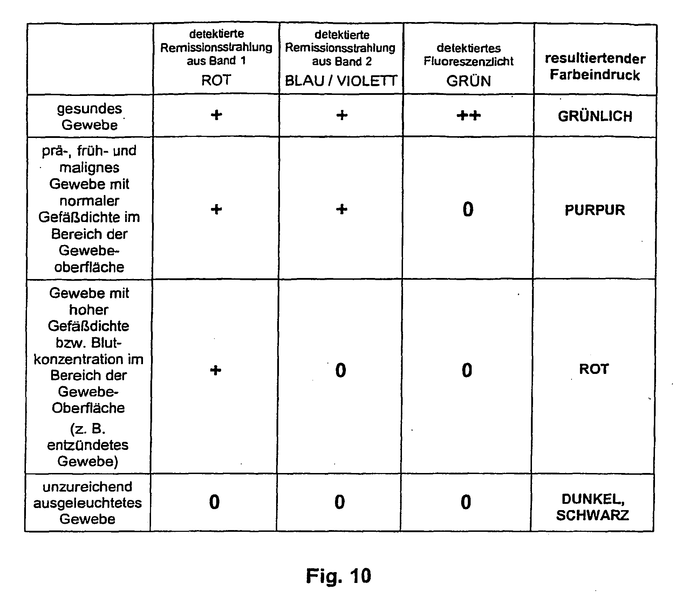

Fig. 10 zeigt in der Übersicht und in Tabellenform die Farbbeiträge für die diversen Gewebezustände und Bildsituationen. Dabei bedeuten:
++ hoher Beitrag
+ mittlerer Beitrag
0 geringer / vernachlässigbarer Beitrag

Die letzte Spalte der Tabelle gibt den jeweils resultierenden Farbeindruck wider, wobei vorausgesetzt wird, dass keine Farbtransformationen, beispielsweise mittels Bildverarbeitung, vorgenommen werden. Werden solche Farbtransformationen vorgenommen, läßt sich das Gewebe in nochmals völlig anderen Farben darstellen. Dies kann beispielsweise dann sinnvoll sein, wenn man potenziellen Sehschwächen des Anwenders begegnen will.

Denkbar ist auch, dass auf das menschliche Auge oder die Bildaufnahmeeinheit im Arbeitsmodus der DAFE von der Lichtquelle bereitgestellte und am Gewebe remittierte Strahlung aus weiteren spektralen Bändern bzw. weiteren Gruppen von spektralen Bändern übertragbar ist, die dadurch gekennzeichnet sind, dass sich dass Remissionsverhalten von weiteren entweder oben namentlich aufgeführten Gewebezuständen und/oder namentlich nicht genannten Gewebezuständen für Strahlung aus diesen spektralen Bändern bzw. Gruppen von spektralen Bändern unterscheidet. Dadurch ist eine weiter verbesserte Gewebedifferenzierung möglich.

Literatur:
[1] M. Zellweger, P. Grosjean, D. Goujon, P. Monnier, H. van den Bergh, G. Wagnières: In vivo autofluorescence spectroscopy of human bronchial tissue to optimize the detection and imaging of early cancers, Journal of Biomedical Optics, 6(1), pp 41-51, 2001
[2] M. Leonhard: New Incoherent Autofluorescence/Fluorescence System for Early Detection of Lung Cancer, Diagnostic and Therapeutic Endoscopy, Vol. 5, pp. 71-75, 1999

## Patentansprüche

1. Vorrichtung zur bildgebenden Diagnose von Gewebe (1) unter wahlweiser Anwendung von zwei Diagnosemethoden, nämlich einem Arbeitsmodus zur diagnostischen Weißlicht-Endoskopie DWLE und einem Arbeitsmodus zur diagnostischen Autofluoreszenz-Endoskopie DAFE, mit einer Lichtquelle (2), deren Licht über einen Lichtleiter (3) zum Gewebe (1) geleitet wird oder deren Leuchtmittel selbst in unmittelbare Nähe des Gewebe geführt werden kann, und mit einer Bildübertragungseinheit (4), die entweder mit dem menschlichen Auge oder alternativ mit einer Bildaufnahmeeinheit (5) in Verbindung steht, welche wiederum an eine Bildverarbeitungseinheit (6) angeschlossen ist, über welche ein Monitor (7) mit einem Bildsignal versorgt wird, wobei auf das menschliche Auge oder auf die Bildaufnahmeeinheit (5) im Arbeitsmodus der DAFE neben dem im Gewebe (1) erzeugten Fluoreszenzlicht oder Teilen des im Gewebe (1) erzeugten Fluoreszenzlichts zusätzlich von der Lichtquelle (2) bereitgestellte und am Gewebe (1) remittierte Strahlung aus einem ersten und einem zweiten spektralen Band übertragen wird, **dadurch gekennzeichnet, dass** die Strahlung aus dem ersten Band in einem schmalen Wellenlängenbereich um 670 +/- 10 nm liegt, so dass das Remissionsverhalten des Gewebes von den unterschiedlichen Gewebezuständen unabhängig oder aber zumindest in erster Näherung unabhängig ist, und dass die Strahlung aus dem zweiten Band in einem schmalen Wellenlängenbereicht um 415 nm bei einer Halbwertsbreite von 10 nm liegt, so dass sich Gewebe mit einer vergleichsweise hohen Gefäßdichte bzw. Blutkonzentration hinsichtlich der Remission von Strahlung aus diesem zweiten Band anders verhält als Gewebe mit einer im Vergleich dazu geringeren Gefäßdichte bzw. Blutkonzentration und/oder als Gewebe mit einer ähnlich hohen Gefäßdichte bzw. Blutkonzentration, welche aber in einem anderen Abstand zur Gewebeoberfläche auftritt.

2. Vorrichtung nach Anspruch 1, **dadurch gekennzeichnet, dass** sowohl die Strahlung aus dem ersten spektralen Band als auch die Strahlung aus dem zweiten spektralen Band mit die Intensität der jeweiligen Strahlung manipulierenden Komponenten der Vorrichtung so abgestimmt ist, dass die jeweils am Gewebe remittierten und von der Bildaufnahmeeinheit detektierten maximalen Anteile aus den beiden Bändern bzw. den beiden Gruppen von Bändern in der Größenordnung der detektierten Fluoreszenzstrahlung von gesundem Gewebe liegen.

3. Vorrichtung nach Anspruch 1 oder 2, **dadurch gekennzeichnet, dass** die Gewebe-Fluoreszenzstrahlung und die remittierten Strahlungen aus den beiden spektralen Bändern zeitgleich übertragbar und detektierbar sind.

4. Vorrichtung nach einem der Ansprüche 1 oder 2, **dadurch gekennzeichnet, dass** die Gewebe-Fluoreszenzstrahlung und die remittierten Strahlungen aus den beiden spektralen Bändern sequentiell, d. h. zeitlich nacheinander, übertragbar und detektierbar sind.

5. Vorrichtung nach einem der vorangehenden Ansprüche, **dadurch gekennzeichnet, dass** die Bildübertragungseinheit (4) aus dem Objektiv eines Chipendoskops, die Bildaufnahmeeinheit (5) aus dem Sensorsystem eines Chipendoskops und die Bildverarbeitungseinheit (6) aus dem zugehörigen Controller besteht.

6. Vorrichtung nach einem der Ansprüche 1 bis 4, **dadurch gekennzeichnet, dass** die Bildübertragungseinheit (4) aus dem Objektiv, aus dem Linsensystem oder Bildfaserbündel und dem Okular eines Endoskops sowie aus dem Objektiv einer Kamera mit Sensorsystem besteht und dass die Bildaufnahmeeinheit (5) aus dem Sensorsystem der Kamera und die Bildverarbeitungseinheit (6) aus dem zugehörigen Treiber besteht.

7. Vorrichtung nach einem der vorangehenden Ansprüche, **dadurch gekennzeichnet, dass** die Lichtquelle (2) mindestens ein inkohärent und spektral breitbandig emittierendes Leuchtmittel (8) aufweist.

8. Vorrichtung nach einem der vorangehenden Ansprüche, **dadurch gekennzeichnet, dass** die Lichtquelle (2) eines oder mehrere Leuchtmittel (8) umfasst und dass eines oder mehrere oder alle dieser Leuchtmittel in einem Endoskop untergebracht sind.

## Claims

1. A device for the picture-providing diagnosis of tissue (1) amid the selective application of two diagnosis methods, specifically an operating mode for diagnostic white light endoscopy DWLE and an operating mode for diagnostic auto-fluorescence endoscopy DAFE, with a light source (2), whose light is led to the tissue (1) via a fibre optic (3) or whose illumination means itself may be led up to the direct vicinity of the tissue, and with a picture transmission unit (4) which is either in connection with the human eye or alternatively with a picture recording unit (5), which in turn is connected to a picture processing unit (6), via which a monitor (7) is supplied with a picture signal, wherein in the operating mode of DAFE, apart from the fluorescence light produced in the tissue (1) or parts of the fluorescence light produced in the tissue (1), radiation from a first and a second spectral band, which is additionally made available by the light source (2) and which is reflected at the tissue (1) is transmitted to the human eye or the picture recording unit (5), **characterised in that** the radiation from the first band lies in a narrow wavelength region around 670 +/-10 nm, so that the reflectance behaviour of the tissue is independent of the different tissue conditions or is however at least independent to a first approximation, and that the radiation from the second band lies in a narrow wavelength region around 415 nm with a half-value width of 10 nm, so that tissue with a comparatively high vessel density or blood concentration, with respect to the reflectance of radiation from this second band behaves differently to tissue with a vessel density or blood concentration which is lower compared to this and/or to tissue with a similarly high vessel density or blood concentration, but which occurs at a different distance to the tissue surface.

2. A device according to claim 1, **characterised in that** the radiation from the first spectral band as well as the radiation from the second spectral band is matched to the intensity of the respective radiation of manipulating components, such that the maximal shares from the two bands or the two groups of bands, which in each case are reflected at the tissue and detected by the picture recording unit, lie in the order of magnitude of the detected fluorescence radiation of healthy tissue.

3. A device according to one of the claims 1 or 2, **characterised in that** the tissue fluorescence radiation and the reflected radiation from the two spectral bands may be transmitted and detected simultaneously.

4. A device according to one of the claims 1 or 2, **characterised in that** the tissue fluorescence radiation and the reflected radiation from the two spectral bands may be transmitted and detected sequentially, i.e. after one another with respect to time.

5. A device according to one of the preceding claims, **characterised in that** the picture transmission unit (4) consists of the objective of a chip endoscope, the picture recording unit (5) of the sensor system of a chip endoscope, and the picture processing unit (6) of the associated controller.

6. A device according to one of the claims 1 to 4, **characterised in that** the picture transmission unit (4) consists of the objective, of the lens system or picture fibre bundle and of the eyepiece of an endoscope as well as of the objective of a camera with a sensor system, and that the picture recording unit (5) consists of the sensor system of the camera, and the picture processing unit (6) of the associated driver.

7. A device according to one of the preceding claims, **characterised in that** the light source (2) comprises at least one illumination means (8) emitting in an incoherent and spectrally broad-banded manner.

8. A device according to one of the preceding claims, **characterised in that** the light source (2) comprises one or more illumination means and that one or more or all of these illumination means are accommodated in an endoscope.

## Revendications

1. Dispositif de diagnostic de tissu par formation d'image (1) en utilisant au choix deux méthodes de diagnostic, à savoir un mode de travail permettant une endoscopie diagnostique en lumière blanche (DWLE) et un mode de travail permettant une endoscopie diagnostique par autofluorescence (DAFE), comportant une source lumineuse (2) dont la lumière est dirigée vers le tissu (1) via une fibre optique (3) ou dont le moyen d'éclairage lui-même peut être dirigé à proximité immédiate du tissu, et une unité de transmission des images (4) qui est en relation soit avec l'oeil humain soit comme alternative avec une unité d'enregistrement des images (5) qui est à son tour raccordée à une unité de traitement des images (6), via laquelle un écran (7) est alimenté en signal d'image, où sur l'oeil humain ou sur l'unité d'enregistrement des images (5) en mode de travail de la DAFE est transmis, en plus de la lumière de fluorescence générée dans le tissu (1) ou de parties de la lumière de fluorescence générée dans le tissu (1), un rayonnement, mis à disposition par la source lumineuse (2) et réfléchi au niveau du tissu (1), d'une première et d'une seconde bandes spectrales, **caractérisé en ce que** le rayonnement de la première bande se situe dans une plage de longueurs d'ondes étroite autour de 670 ± 10 nm, si bien que le comportement de réflectance du tissu est indépendant des différents états du tissu ou cependant au moins indépendant en première approximation, et **en ce que** le rayonnement de la seconde bande se situe dans une plage de longueurs d'ondes étroite autour de 415 nm avec une largeur à mi-hauteur de 10 nm, si bien que le tissu présentant une densité de vaisseaux ou une concentration sanguine comparativement élevée se comporte, pour ce qui est de la réflectance du rayonnement de la seconde bande, de façon différente du tissu présentant une densité de vaisseaux ou une concentration sanguine comparativement plus faible et/ou que le tissu présentant une densité de vaisseaux ou une concentration sanguine aussi élevée, mais qui se trouve à une autre distance de la surface du tissu.

2. Dispositif selon la revendication 1, **caractérisé en ce que** tant le rayonnement de la première bande spectrale que le rayonnement de la seconde bande spectrale sont accordés avec les composants du dispositif manipulant l'intensité du rayonnement respectif de façon telle que les parties maximum, réfléchies respectivement au niveau du tissu et détectées par l'unité d'enregistrement des images, des deux bandes ou des deux groupes de bandes sont dans l'ordre de grandeur du rayonnement de fluorescence du tissu sain détecté.

3. Dispositif selon la revendication 1 ou 2, **caractérisé en ce que** le rayonnement de fluorescence du tissu et les rayonnements réfléchis des deux bandes spectrales peuvent être transmis et détectés simultanément.

4. Dispositif selon l'une des revendications 1 ou 2, **caractérisé en ce que** le rayonnement de fluorescence du tissu et les rayonnements réfléchis des deux bandes spectrales peuvent être transmis et détectés séquentiellement, à savoir l'un après l'autre dans le temps.

5. Dispositif selon l'une des revendications précédentes, **caractérisé en ce que** l'unité de transmission des images (4) se compose de l'objectif d'un endoscope à puce, l'unité d'enregistrement des images (5) du système de capteur d'un endoscope à puce et l'unité de traitement des images (6) du contrôleur associé.

6. Dispositif selon l'une des revendications 1 à 4, **caractérisé en ce que** l'unité de transmission des images (4) se compose de l'objectif, de l'optique ou du faisceau de fibres d'image et de l'oculaire d'un endoscope, ainsi que de l'objectif de caméra avec système de capteur, et **en ce que** l'unité d'enregistrement des images (5) se compose du système de capteur de la caméra, et l'unité de traitement des images (6) du pilote correspondant.

7. Dispositif selon l'une des revendications précédentes, **caractérisé en ce que** la source lumineuse (2) présente au moins un moyen d'éclairage (8) à émission incohérente en large bande spectrale.

8. Dispositif selon l'une des revendications précédentes, **caractérisé en ce que** la source lumineuse (2) comprend un ou plusieurs moyens d'éclairage (8) et **en ce qu'**un ou plusieurs ou tous ces moyens d'éclairage sont logés dans un endoscope.
